# EUROPEAN PATENT SPECIFICATION

(11) **EP 2 922 457 B1**
(45) Date of publication and mention of the grant of the patent: **05.12.2018**
(21) Application number: 13782891.9
(22) Date of filing: 09.10.2013
(51) Int. Cl.: A61B 1/00, A61B 1/045, G02B 23/24

(54) **ENDOSCOPE COMPRISING AN IMAGE SENSOR ASSEMBLY WITH CALIBRATION MEMORY**
ENDOSKOP UMFASSEND EINE BILDSENSORBAUGRUPPE MIT KALIBRIERSPEICHER
ENDOSCOPE COMPRENANT UN MODULE DE DÉTECTEUR À FORMATION D'IMAGES AVEC UNE MÉMOIRE D'ÉTALONNAGE

(30) Priority: 22.11.2012 US 201213684180
(43) Date of publication of application: 30.09.2015
(73) Proprietor: Gyrus Acmi Inc., Southborough, MA 01772 (US)
(72) Inventor: BLUMENZWEIG, Arie, 42243 Netanya (IL); FINKMAN, Shai, 34861 Haifa (IL); WOLF, Stuart, 20600 Yokneam (IL)
(74) Representative: Gunzelmann, Rainer
(86) International application number: PCT/US2013/063957
(87) International publication number: WO 2014/081512

(56) References cited:
- EP-A1- 2 689 717
- JP-A- 2012 075 516
- US-A- 5 896 166
- US-A1- 2008 108 869
- US-A1- 2008 165 360
- US-A1- 2011 237 885
- US-A1- 2012 127 294
- YAP-PENG TAN ET AL: "A robust sequential approach for the detection of defective pixels in an image sensor", ICASSP, IEEE INTERNATIONAL CONFERENCE ON ACOUSTICS, SPEECH AND SIGNAL PROCESSING - PROCEEDINGS 1999 IEEE, IEEE, vol. 4, 15 March 1999 (1999-03-15), pages 2239-2242, XP010327884, DOI: 10.1109/ICASSP.1999.758382 ISBN: 978-0-7803-5041-0

## Description

### FIELD OF THE INVENTION

The present invention relates generally to imaging devices, and specifically to the construction of an imaging device such as an endoscope.

### BACKGROUND OF THE INVENTION

Many imaging devices, such as endoscopes, are inherently limited in size, and in many cases a small imaging device is advantageous. In the case of an endoscope, the smaller the dimensions of the endoscope, the less the impact on a patient being investigated. Consequently, any system which enables an endoscope to operate efficiently, while maintaining its small dimensions, would be beneficial.

In the fabrication of a pixel-based imaging device, there may typically be one or more "bad" pixels, or other factors such as the presence of fixed pattern noise, which affect the quality of operation of the device. Such factors may typically be compensated for by calibrating the device.

U. S. Patent 6,417,885, to Suzuki et al., describes a solid-state image pickup device with a separable circuit board. The disclosure states that an image pickup section incorporates a solid-state image pickup element chip, and that electronic parts for driving or controlling the image pickup section are mounted on a circuit board.

U. S. Patent Application 2009/0292169, to Mitani et al., describes an electronic endoscope apparatus. The apparatus is stated to include an image pickup apparatus mounted at a distal end portion of an insertion portion, and an operation portion provided "consecutively" at the insertion portion.

Japanese Patent Publication 07-327923, to Saito et al., describes an endoscope system. The system is stated to be easy to assemble and to be safe for medical use. The disclosure further states that these qualities are accomplished by assuring the cleanliness of an endoscope side connector arranged on a clean area side of a junction cable.

Japanese Patent Publication 05-154098, to Matsumoto, describes a signal processing circuit for an electronic endoscope apparatus. The apparatus is claimed to achieve a simplification of circuitry and as well as ease of handling by using a "correlation double sampling circuit." The circuit is stated to operate effectively, without converting operation timing, even when an electronic endoscope with a varied length is used.

Japanese patent application JP 2012 075516 A discloses defect pixel correction of a distal CCD image sensor by circuitry provided on a circuit board mounted within a connector (28) at the terminal end of the endoscope cable, the electronics comprising a signal processing part (50) connected via a cable with the distal image sensor, an image correction part (52), and a memory (54) storing calibration data. This document does not disclose that the memory and the control circuit are provided on different printed circuit boards connected by a tongue and a mating connector within the handle portion.

### SUMMARY OF THE INVENTION

The present invention provides an endoscope as defined in claim 1.

Typically, the insertion portion detachably connects to the handle portion.

In a disclosed embodiment the calibration data corrects for a defective pixel of the image sensor.

Further aspects of the invention are defined in the dependent claims.

The present invention will be more fully understood from the following detailed description of embodiments taken together with the drawings in which:

### BRIEF DESCRIPTION OF THE DRAWINGS

Fig. 1 is a schematic exploded view of an endoscope, according to an embodiment of the present invention;
Fig. 2 is a schematic assembled view of the endoscope, according to an embodiment of the present invention;
Fig. 3 is a schematic illustration of the endoscope connected to an endoscope control unit, according to an embodiment of the present invention;
Fig. 4 is a schematic illustration of a sensor and a sensor connector, according to an embodiment of the present invention;
Fig. 5 is a schematic illustration of the sensor connector, according to an embodiment of the present invention;
Fig. 6 is a schematic diagram of an endoscope control circuit, according to an embodiment of the present invention;
Fig. 7 is a schematic illustration of a setup for calibrating a sensor assembly according to an embodiment of the present invention;
Fig. 8 is a flowchart of steps followed for the calibration of the sensor assembly according to an embodiment of the present invention; and
Fig. 9 illustrates a look-up calibration table produced for the sensor assembly according to an embodiment of the present invention.

### DETAILED DESCRIPTION OF EMBODIMENTS

### OVERVIEW

In an embodiment of the present disclosure an endoscope comprises an uncalibrated image sensor, typically in the form of a rectangular array of sensor elements or pixels. The endoscope also comprises a control circuit which is mounted on or in the endoscope, and the control circuit is configured to drive the image sensor, based on a control signal from a processor. The processor is typically incorporated into an endoscope control unit which operates the endoscope.

A connector is connected to the image sensor, typically via a cable, the connector, the cable, and the sensor forming a sensor assembly which is a stand-alone unit. The connector comprises a memory which stores calibration data for the image sensor. The calibration data typically comprises respective correction factors for each of the pixels of the image sensor, and the correction factors may be determined in a calibration process implemented for the sensor assembly.

During operation of the endoscope, the control circuit adjusts the uncalibrated signals from the image sensor, based on the calibration data stored in the memory of the connector, to form a calibrated image signal. The control circuit then transmits the calibrated image signal to the processor, which typically uses the calibrated signal to generate a display on a screen.

Forming a sensor assembly from an uncalibrated image sensor, and incorporating calibration data into a memory separated from the image sensor but which is within the sensor assembly, is an efficient and cost-effective technique for producing a calibrated sensor assembly. Such an assembly generates calibrated image signals from an uncalibrated sensor.

Sensor assemblies as exemplified in embodiments of the present disclosure separate the connector, which has a memory with sensor calibration data stored therein, from the sensor itself. The separation enables the size of the sensor to be minimized, while maintaining the association of the sensor calibration data with the sensor. Minimization of the sensor size is a critical advantage, enabling the section of the endoscope wherein the sensor operates to have a considerably reduced size compared to prior art systems.

### DETAILED DESCRIPTION

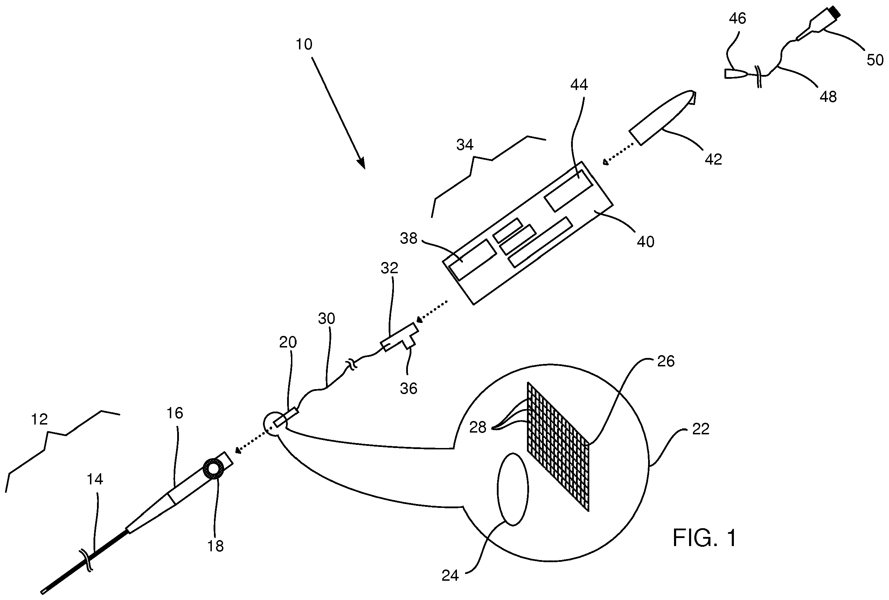

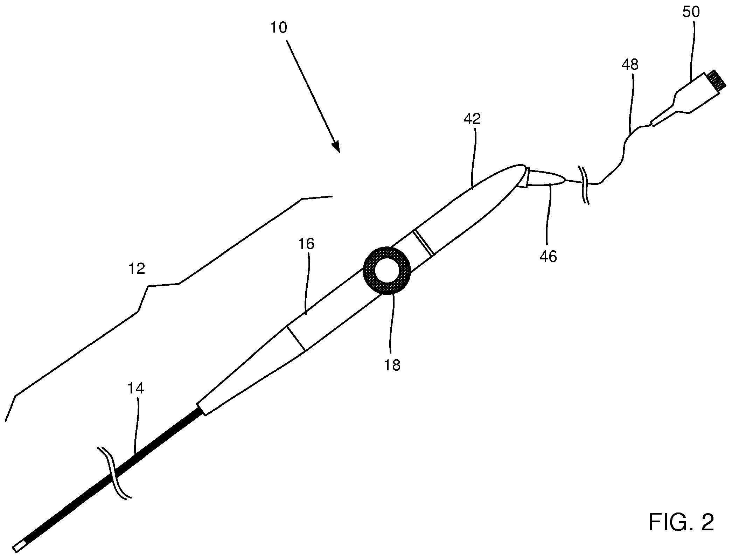

Fig. 1 and Fig. 2 are respectively an exploded view and an assembled view of an endoscope 10, according to an embodiment of the present invention. In Fig. 1 and Fig. 2, and in other figures of the present disclosure, multiple views of different components are illustrated. In the figures, each specific component has the same identifying numeral.

Endoscope 10 comprises a tubular insertion portion 12, which is formed as a small diameter tubular member 14 connected to a tubular member receptacle 16. Tubular member 14 is implemented to have an external diameter allowing it to be inserted into a body cavity, such as an abdomen, of a patient. A typical external diameter of member 14 is approximately 5 mm, and a typical internal diameter of the member is less than 5 mm. Typically, receptacle 16 has external dimensions significantly larger than the external diameter of member 14, and comprises one or more controls 18 for the endoscope. The insertion of member 14 may be via a trocar, normally used during an investigative procedure on the patient. For simplicity, neither the trocar nor the patient are shown in Figs. 1 or 2.

An image sensor 20, components of which are shown in more detail in an inset 22, is mounted at the distal end of tubular member 14. Typically, sensor 20 is approximately cylindrical or is in the form of a rectangular parallelepiped, having a largest dimension smaller than the internal diameter of member 14. Image sensor 20 is uncalibrated, and comprises an optical assembly 24, typically formed of one or more lenses, which focuses light incident on the assembly from a scene being viewed by the sensor. Assembly 24 focuses the incident light (typically light returning from light that has been projected onto the abdomen wall) onto an array 26 of sensing elements 28, which are also referred to herein as pixels 28. Array 26 typically comprises CCD (charge coupled device) and/or CMOS (complementary metal oxide semiconductor) pixels 28 arranged on a die in a rectangular grid.

Image sensor 20 is connected by a sensor cable 30 to a sensor connector 32. Cable 30 transfers signals, including video, control, timing, and power signals, between the sensor connector and the image sensor. Typically, cable 30 comprises one or more electrical conductors for transferring the signals, although in some embodiments at least some of the signals may be transferred using one or more optical fibers located within the cable. In one embodiment sensor connector 32 is formed as a printed circuit board (PCB) having a thickness of approximately 0.3 mm.

Sensor connector 32 is configured to connect to an endoscope control circuit 34. In one embodiment, the sensor connector comprises a tongue 36, and control circuit 34 comprises a first connector element 38 which is configured as a zero insertion force (ZIF) socket mating with the tongue. In a disclosed embodiment control circuit 34, described in more detail below, is mounted on a PCB 40.

PCB 40, together with the control circuit mounted on the PCB, is configured to fit into a handle portion 42 of the endoscope, and the PCB is also referred to herein as handle board 40. Control circuit 34 includes a second connector element 44 which is configured to mate with a first termination 46 of an endoscope control cable 48. A second termination 50 connects to an endoscope control unit, described in more detail below.

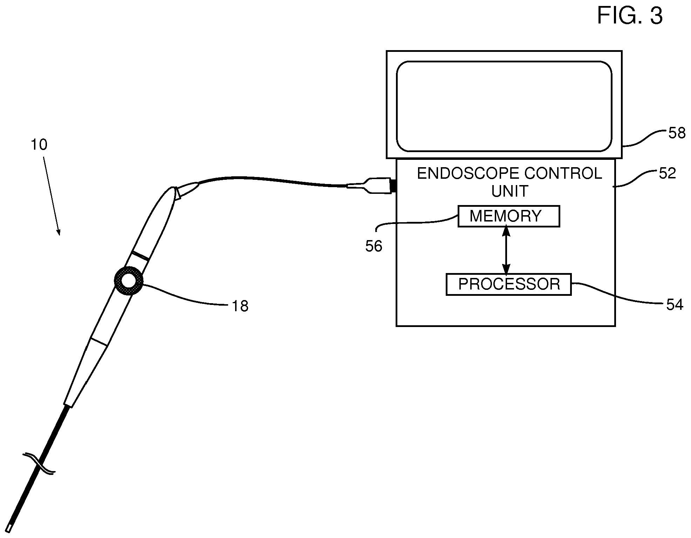

Fig. 3 is a schematic illustration of endoscope 10 connected to an endoscope control unit 52, according to an embodiment of the present invention. Unit 52 provides overall management of endoscope 10, so that in addition to controls 18, an operator of the endoscope, typically a physician, is able to set parameters of functions of the endoscope via the unit. The functions include, *inter alia,* adjusting the illumination used by the endoscope in viewing the scene imaged by sensor 20. The operator typically communicates with unit 52 via a pointing device and/or a keypad (neither are shown in the figure).

Unit 52 comprises a processor 54 communicating with a memory 56, and software for operation of the endoscope is stored in the memory. Results of operations performed by processor 54 may be presented to the operator of system 10 on a screen 58, which typically displays an image of the scene being imaged by sensor 20. The software used by processor 54 may be downloaded to the processor in electronic form, over a network, for example, or it may, alternatively or additionally, be provided and/or stored on non-transitory tangible media, such as magnetic, optical, or electronic memory.

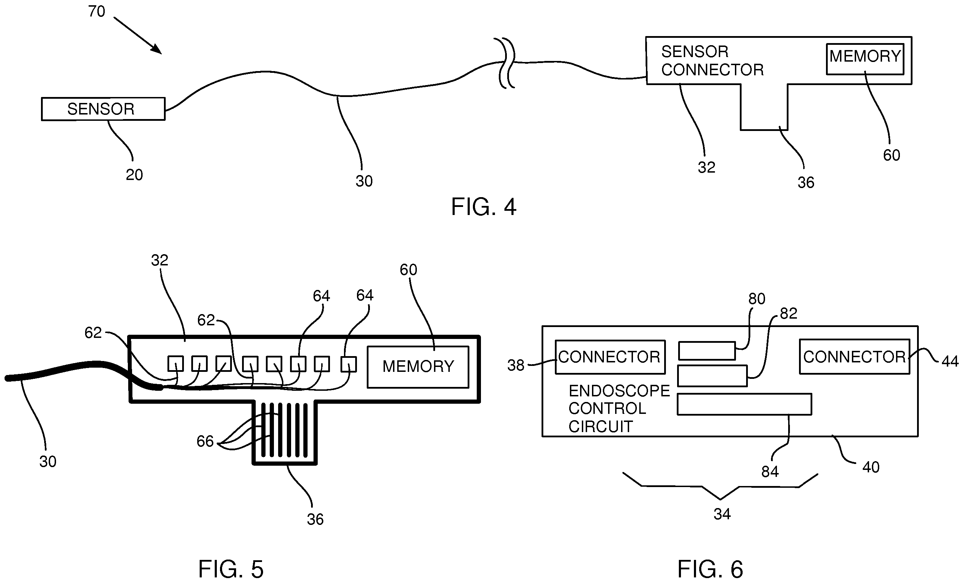

Fig. 4 is a schematic illustration of sensor 20 and sensor connector 32, and Fig. 5 is a schematic illustration of the sensor connector, according to embodiments of the present invention. A non-volatile sensor calibration memory 60 is attached to sensor connector 32, typically by soldering. The function of memory 60 is described below. Individual wires 62 of cable 30, exiting from a proximal end of the cable, are connected, typically by soldering, to pads 64 on connector 32. The wires are also connected, at a distal end of cable 30, to sensor 20. In addition, conductive terminals 66 are formed on tongue 36, the conductive terminals being configured to mate with conductors in first conductor element 38 (of control circuit 34). As is illustrated by Fig. 4, the combination of sensor 20, cable 30, and sensor connector 32 forms a sensor assembly 70. After a calibration process described below, sensor assembly 70 may be considered as a single "stand-alone" calibrated sensor unit which may be used in endoscope 10, or in other endoscopes similar in construction to endoscope 10.

Fig. 6 is a schematic diagram of endoscope control circuit 34, according to an embodiment of the present invention. Circuit 34 is mounted on handle board 40, and the circuit on its board is typically installed in handle portion 42. Circuit 34 acts as a local controller for functions used by the endoscope, the functions including driving sensor 20 in response to a control signal from endoscope control unit 52, and transferring a calibrated image signal, generated from sensor assembly 70, to endoscope control unit 52. Other functions performed by circuit 34 include controlling illumination units used by the endoscope.

In one embodiment circuit 34 comprises a field programmable gate array (FPGA) 80, a non-volatile memory 82, and an electronic sub-circuit 84. FPGA 80 may be configured to convert data from sensor 20 to a format suitable for endoscope control unit 52, for example by converting serial data to parallel data or to a low voltage differential signaling (LVDS) format. FPGA 80 may also be configured to transfer communications, such as I²C communications, between endoscope control unit 52, sensor 20, and buttons or other controls on handle portion 42 that may be used by an operator of endoscope 10 in controlling the endoscope. Non-volatile memory 82 may store data enabling endoscope control unit 52 to recognize which particular type of endoscope is being used. Typically, the FPGA receives an uncalibrated image signal from sensor 20, generates a calibrated signal using calibration data stored in memory 60, and transfers the calibrated signal to the control unit. The calibration data stored in memory 60 is described in more detail below. Electronic sub-circuit 84 facilitates the transfer of data to and from FPGA 80.

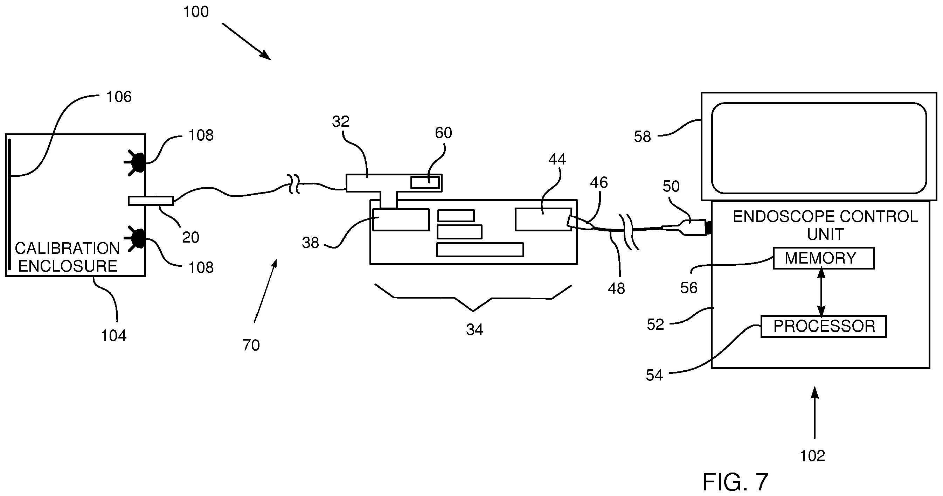

Fig. 7 is a schematic illustration of a setup 100 for calibrating sensor assembly 70. Setup 100 is typically located in a factory producing assembly 70. Typically, apart from random noise such as thermal noise, there are variations in response of pixels 28 of array 26, and in the signals derived from the pixels. The variations are caused, for example, by differences in the dimensions of the pixels and variations in the composition of the pixel constituents. The variations may also be from differences in amplification factors, typically from pre-amplifiers located within sensor 20, that are applied to the signals generated by the pixels to form amplified signals output by the sensor. While all these variations are usually small, one or more of them may be present in array 26 and/or sensor 20, and may result in the pixel response variations referred to above. In addition to these variations, some arrays may comprise pixels that are substantially unresponsive to illumination, typically because of a defect in the manufacturing process of the array. Such unresponsive pixels are herein termed defective pixels.

Calibration setup 100 is operated in the factory by a calibration control unit 102, which typically presents details of the calibration process to a professional performing the calibration on a display unit. Herein, by way of example, it is assumed that endoscope control unit 52 has been configured as calibration control unit 102, so that processor 54 of the endoscope control unit acts together with memory 56 as an overall controller for the calibration, including presenting calibration details on screen 58.

A calibration enclosure 104 is configured to receive sensor 20, so that the sensor is fixedly mounted to the unit. Enclosure 104 is configured to allow preset displays to be presented to sensor 20 on a screen 106, so that the sensor is able to acquire test images of the displays for calibration. Screen 106 may comprise a luminescent monitor, such as a flat screen LCD video monitor. Alternatively, screen 106 may be passive, and displays on the screen may be formed on the screen from illuminators 108 in enclosure 104. Further alternatively, the displays may be in the form of a target such as a Gretag-Macbeth color chart or a uniform grayscale chart, which could be paper or backlit. The displays presented on screen 106, i.e., the intensities and colors, or RGB (red, green, blue) levels, of individual regions of the screen, are under overall management of control unit 102.

In setup 100 connector 32 is inserted into first connector element 38 of control circuit 34, and the circuit is connected to calibration control unit 102 by cable 48. It will be appreciated that the arrangement of electronic components in setup 100 is substantially the same as the arrangement of the components internally in endoscope 10, so that in some embodiments, rather than assembly 70 being calibrated separately from its endoscope, the assembly may be calibrated while it is in its endoscope. In this case, tubular member 14, rather than sensor 20, is inserted into calibration enclosure 104.

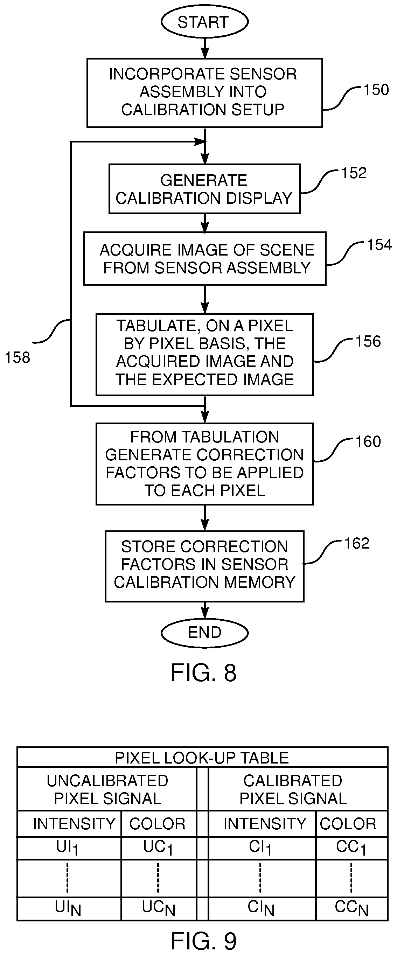

Fig. 8 is a flowchart of steps followed for the calibration of sensor assembly 70, and Fig. 9 illustrates a look-up calibration table produced for the assembly. In the description of the flowchart, it is assumed that screen 106 comprises a luminescent monitor, as described above, which is operated by calibration control unit 102. In addition, it is assumed that the calibration is performed without sensor assembly 70 being installed in an endoscope. Those having ordinary skill in the art will be able to adapt the description for other types of screens, and for the case of the sensor assembly being installed in an endoscope.

In an initial step 150, sensor 20 of the sensor assembly is inserted into calibration enclosure 104, and the assembly is connected, as illustrated in Fig. 7, via circuit 34 to calibration control unit 102.

In a scene generation step 152, the calibration control unit creates a display on screen 106. Typically, the display generated comprises a single color with equal intensities being emitted from each area of the whole screen.

In an image acquisition step 154, the calibration control unit operates sensor 20, via a control signal transmitted to control circuit 34, which in turn drives the sensor. The sensor acquires an image of the scene displayed on screen 106 by scanning signals generated by pixels 28 of the sensor, and the image of the scanned signals returns to the control unit via control circuit 34. The returned image, i.e., the image received by the control unit, comprises respective uncalibrated signals originating from respective pixels 28 of array 26.

In a tabulation step 156, the processor of calibration control unit 102 tabulates, on a pixel by pixel basis, the uncalibrated signal from each pixel 28 together with an expected pixel signal. The expected pixel signal comprises levels that the control unit expects to receive back from the display created by the unit on screen 106. The expected pixel signal typically comprises an expected intensity level and an expected color value. Similarly, the uncalibrated pixel signal comprises an actual measured intensity level and an actual measured color value. Typically, in order to cancel out random noise effects, such as thermal noise, the measured intensity level and the measured color value (for each pixel) are acquired for a number of scans of sensor 20 over time, and the measured levels are averaged.

For each pixel, the tabulation, i.e., the measured and expected intensity levels and the measured and expected color values, may be stored in the memory of calibration control unit 102.

As illustrated by a line 158, the calibration control unit reiterates steps 152, 154, and 156. In each reiteration, in step 152 the control unit creates a different display on screen 106, and in step 156, the values of the measured and expected intensity levels and the measured and expected color values are stored in memory.

A number of iterations, N, required for a satisfactory calibration of sensor assembly 70, and the different displays generated on screen 106 for each iteration, may be determined by an operator of the calibration process without undue experimentation. In one embodiment, three different intensity levels, for each of nine different colors (red, green, blue, magenta, cyan, yellow, white, black, grey), i.e., a total of 27 displays, are created on screen 106. Larger numbers of displays typically provide greater accuracy for the calibration.

In a correction factor step 160, the calibration control unit uses the tabulation generated in step 156 to generate a correction factor for each pixel 28 of array 26. Typically, for each pixel the correction factor is incorporated into a look-up table for the pixel.

Fig. 9 illustrates a look-up table for a given pixel. As illustrated, the table has sets of uncalibrated intensity signal levels, UIₙ, and uncalibrated color signal levels, UCₙ, where n is an integer between 1 and N, the number of iterations. For each set of uncalibrated intensity and color signal levels, there is a corresponding set of calibrated intensity, CIₙ, and color signal, CCₙ, levels. The set of calibrated levels corresponds to the expected intensity and color levels, described above with reference to step 156.

In a memory storage step 162, the correction factors for each pixel 28, typically corresponding to a look-up table as illustrated in Fig. 9, are stored in sensor calibration memory 60 of sensor connector 32. The flowchart then ends.

In the above description the correction factors for each pixel 28 have been assumed to be in the form of a look-up table. As necessary, during operation of endoscope 10 with sensor assembly 70 (after calibration as described with reference to the flowchart of Fig. 8) endoscope control unit 52 may apply interpolation and/or extrapolation to accommodate received uncalibrated signal levels not in the table. However, there is no necessity that the correction factors be in the form of a look-up table, and the factors may be in any convenient form, such as equations relating the calibrated values to measured uncalibrated signal levels. All such forms of the correction factors are assumed to be comprised within the scope of the present invention.

In some embodiments the correction factors for a given pixel are dependent on signals received by neighboring pixels, including nearest neighbor pixels, next-neighbor pixels, and/or pixels further these neighboring pixels from the given pixel. Such correction factors may be generated, *mutatis mutandis,* using the flowchart of Fig. 8, and are assumed to be comprised within the scope of the present invention.

In some embodiments, one or more pixels 28 may be defective, giving substantially no response when illuminated. Such defective pixels are also termed "bad" pixels. A correction factor for a defective pixel may also be generated using the flowchart of Fig. 8, *mutatis mutandis,* and typically assumes that the calibrated signal for the pixel is an average of the calibrated signals for its nearest neighbor pixels.

Once the correction factors have been stored as described above, sensor assembly 70 may be incorporated into endoscope 10, and endoscope control unit 52 may be configured to use the corrected values generated by the factors in generating the display on screen 58.

It will be understood that implementation of sensor assembly 70, as described above, may be performed using a sensor 20 having pixels 28. Production of sensor assembly 70 with a sensor, as described hereinabove, allows for the use of a significantly smaller sensor (compared to prior art systems) in endoscope 10, with no reduction of efficiency of operation of the endoscope. Embodiments of the present invention enable sensor 20 to be smaller than prior art systems by incorporating calibration data for the sensor into memory 60 of sensor connector 32.

It will be appreciated that the embodiments described above are cited by way of example, and that the present invention is not limited to what has been particularly shown and described hereinabove.

## Claims

1. An endoscope (10), comprising:
a handle portion (42) and an insertion portion (12) extending from the handle portion (42);
a control circuit (34) mounted on a handle printed circuit board (40), which is disposed within the handle portion (42) and comprises a first connector element (38); and
a sensor assembly (70) comprising:
an image sensor (20) mounted at a distal end of the insertion portion (12) and configured to generate an image signal;
a sensor connector (32) formed as a printed circuit board and comprising a tongue (36) configured to mate with the first connector element (38) and a non-volatile sensor calibration memory (60) storing calibration data with respect to the image sensor; and
a cable (30) passing through the insertion portion (12) and connecting the image sensor (20) to the sensor connector (32), wherein the control circuit is configured to drive the image sensor based on a control signal from a processor (52), to adjust the image signal from the image sensor to form a calibrated image signal, based on the calibration data stored in the memory, and to transmit the calibrated image signal to the processor.

2. The endoscope (10) according to claim 1, wherein the insertion portion (12) is detachably connected to the handle portion (42).

3. The endoscope (10) according to claim 1, wherein the calibration data is suitable for correcting for a defective pixel of the image sensor (20).

4. The endoscope (10) according to claim 1, wherein the sensor calibration data is suitable for correcting a color signal generated by the image sensor (20).

5. The endoscope (10) according to claim 1, wherein the printed circuit board has a thickness of approximately 0,3 mm, and the first connector element (38) is configured as a zero insertion force (ZIF) socket.

## Patentansprüche

1. Endoskop (10), umfassend:
ein Handhabungsteil (42) und ein Einführungsteil (12), welches sich von dem Handhabungsteil (42) erstreckt;
eine Steuerschaltung (34), die auf einer gedruckten Handhabungsschaltplatine (40) montiert ist, welche in dem Handhabungsteil (42) angeordnet ist und ein erstes Verbindungselement (38) umfasst; und
eine Sensoranordnung (70) umfassend:
einen Bildsensor (20), welcher an einem distalen Ende des Einführungsteils (12) montiert ist und welcher konfiguriert ist, um ein Bildsignal zu erzeugen;
einen Sensorverbinder (32), der als eine gedruckte Schaltplatine ausgebildet ist und der eine Zunge (36), welche konfiguriert ist, um sich mit dem ersten Verbindungselement (38) zu verbinden, umfasst und einen nicht flüchtigen Sensorkalibrierungsspeicher (60), welcher Kalibrierungsdaten hinsichtlich des Bildsensors speichert;
ein Kabel (30), welches durch das Einführungsteil (12) verläuft und welches den Bildsensor (20) mit dem Sensorverbinder (32) verbindet, wobei die Steuerschaltung konfiguriert ist, um den Bildsensor basierend auf einem Kontrollsignal von einem Prozessor (52) zu steuern, um das Bildsignal von dem Bildsensor anzupassen zum Bilden eines kalibrierten Bildsignals basierend auf den in dem Speicher gespeicherten Kalibrierungsdaten, und um das kalibrierte Bildsignal zu dem Prozessor zu senden.

2. Endoskop (10) gemäß Anspruch 1, wobei das Einführungsteil (12) trennbar mit dem Handhabungsteil (42) verbunden ist.

3. Endoskop (10) gemäß Anspruch 1, wobei die Kalibrierungsdaten zum Korrigieren eines fehlerhaften Pixels des Bildsensors (20) geeignet sind.

4. Endoskop (10) gemäß Anspruch 1, wobei die Sensorkalibrierungsdaten zum Korrigieren eines Farbsignals, welches von den Bildsensor (20) erzeugt wurde, geeignet sind.

5. Endoskop (10) gemäß Anspruch 1, wobei die gedruckte Schaltplatine eine Dicke von ungefähr 0,3 mm aufweist und das erste Verbindungselement (38) als ein Nullkraftsockel (ZIF) konfiguriert ist.

## Revendications

1. Endoscope (10), comprenant :
une partie de poignée (42) et une partie d'insertion (12) s'étendant à partir de la partie de poignée (42) ;
un circuit de commande (34) monté sur une carte de circuit imprimé (40) de poignée, qui est disposée à l'intérieur de la partie de poignée (42) et comprend un premier élément de connecteur (38) ; et
un ensemble capteur (70) comprenant :
un capteur d'image (20) monté au niveau d'une extrémité distale de la partie d'insertion (12) et configuré pour générer un signal d'image ;
un connecteur de capteur (32) formé sous la forme d'une carte de circuit imprimé et comprenant une languette (36) configurée pour s'accoupler avec le premier élément de connecteur (38) et une mémoire d'étalonnage de capteur non volatile (60) stockant des données d'étalonnage par rapport au capteur d'image ; et
un câble (30) passant à travers la partie d'insertion (12) et connectant le capteur d'image (20) au connecteur de capteur (32), dans lequel le circuit de commande est configuré pour exciter le capteur d'image sur la base d'un signal de commande provenant d'un processeur (52), pour ajuster le signal d'image provenant du capteur d'image pour former un signal d'image étalonné, sur la base des données d'étalonnage mémorisées dans la mémoire, et pour transmettre le signal d'image étalonné au processeur.

2. Endoscope (10) selon la revendication 1, dans lequel la partie d'insertion (12) est connectée de manière détachable à la partie de poignée (42).

3. Endoscope (10) selon la revendication 1, dans lequel les données d'étalonnage sont appropriées pour corriger un pixel défectueux du capteur d'image (20).

4. Endoscope (10) selon la revendication 1, dans lequel les données d'étalonnage de capteur sont appropriées pour corriger un signal de couleur généré par le capteur d'image (20).

5. Endoscope (10) selon la revendication 1, dans lequel la carte de circuit imprimé présente une épaisseur d'approximativement 0,3 mm, et le premier élément de connecteur (38) est configuré sous la forme d'une prise à force d'insertion nulle (ZIF).
